# EUROPEAN PATENT SPECIFICATION

(11) **EP 1 181 917 B1**
(45) Date of publication and mention of the grant of the patent: **06.05.2009**
(21) Application number: 01120232.2
(22) Date of filing: 23.08.2001
(51) Int. Cl.: A61F 13/56, A61F 13/476, A61F 13/15

(54) **Sanitary absorbent article having flaps and an improved adhesive pattern**
Damenbinde mit Befestigungsflügel und einem verbesserten Klebemuster
Serviette hygiénique pourvue de volets et une configuration améliorée d'adhésif

(30) Priority: 25.08.2000 US 648872
(43) Date of publication of application: 27.02.2002
(73) Proprietor: McNEIL-PPC, Inc., Skillman, New Jersey 08558 (US)
(72) Inventor: Endo, Mari, Demarest, NJ 07627 (US)
(74) Representative: Metten, Karl-Heinz

(56) References cited:
- EP-A- 0 025 611
- EP-A- 0 745 368
- EP-A- 0 923 921
- GB-A- 2 119 656
- US-A- 3 463 154
- US-A- 3 672 371

## Description

### FIELD OF THE INVENTION

The present invention relates to disposable absorbent articles, such as sanitary napkins with side flaps and an improved adhesive pattern. More particularly, the invention relates to a sanitary napkin having a central absorbent element and side flaps extending laterally outward from the central absorbent element. An adhesive attachment means is disposed on a garment faceable surface of the central absorbent element in a pattern that permits the sanitary napkin to better conform to the contours of a wearer's body during use.

### BACKGROUND OF THE INVENTION

Sanitary napkins having side flaps are disclosed in the literature and are generally available in the marketplace. Generally, the flaps extend laterally from a central absorbent core and are intended to drape over the edges of the wearer's panties in the crotch region. Thus, the flaps are disposed between the edges of the wearer's panties in the crotch region and the wearer's thighs. Commonly, the flaps are provided with an attachment means for affixing the flaps to the underside of the wearer's panties. The flaps serve at least two purposes. First, the flaps prevent exudates from soiling the edges of the wearer's panties and second, the flaps, when affixed to the underside of the panties, help stabilize the napkin in the undergarment and prevent it shifting out of place.

Sanitary napkins having flaps are disclosed in U.S. Pal. No. 4,687,478, entitled "Shaped Sanitary Napkin With Flaps", to Van Tilburg on Aug. 18, 1987, U.S. Pat. No. 4,608,047, entitled "Sanitary Napkin Attachment Means", to Mattingly on Aug. 26, 1986, U.S. Pat. No. 4,589,876, entitled "Sanitary Napkin", to Van Tilburg on May 20, 1986, U.S. Pat. No. 4,285,343, entitled "Sanitary Napkin", to McNair on Aug. 25, 1981, U.S. Pat. No. 3,397,697, entitled "Disposable Sanitary Shield For Undergarments", to Rickard on Aug. 20, 1968, U.S. Pat. No. 2,787,271, entitled "Sanitary Napkin", to Clark on Apr. 2, 1957 and U.S. Pat. No. 4,900,320, entitled "Sanitary Napkin With Panty Gathering Flaps, to McCoy on Feb. 13, 1990.

Document EP-A-0 923 921 discloses a sanitary napkin for placement inside a wearer's undergarment. The garment facing surface of the sanitary napkin comprises a layer of adhesive to provide means for attaching the napkin to the undergarment, the layer of adhesive comprising at least two zones of adhesive which are applied at differing basis weights.

Document US-A-3 672 371 discloses different methods and arrangements of connecting adhesive layers to the garment facing surface of sanitary napkins in order to allow for secure attaching napkin to the garment in a wearer's undergarment.

### SUMMARY OF THE INVENTION

It is an object of the present invention to provide a novel sanitary absorbent article that permits a center region of the napkin to better conform to a wearer's body when subjected to laterally compressive forces.

In accordance with the present invention, there has been provided a sanitary absorbent article having a central absorbent element with opposite longitudinal sides and opposite transverse ends, the absorbent article including a pair of flexible side flaps, one flap extending laterally outward from each respective longitudinal side of the absorbent element, the flaps being adapted to be folded over a crotch portion of a wearer's undergarment when in use, the absorbent article further including a adhesive attachment means on a garment faceable surface for securing the article to the undergarment, the adhesive attachment means having a pattern including two longitudinally extending strips, one strip being located adjacent to each respective longitudinal side of the article and two laterally extending strips, one strip being located adjacent to each respective transverse end of the article, said longitudinally extending strips and said laterally extending strips substantially surrounding a center region that is incapable of adhesively securing that portion of the garment faceable surface of the article to the wearer's undergarment.

These and other embodiments of the present invention will be more readily apparent when considered in reference to the following description and when taken in conjunction with the accompanying drawings. It is to be understood, however, that the drawings are provided for purposes of illustration only and not as a definition of the boundaries of the invention, for which reference should be made to the appended claims.

### BRIEF DESCRIPTION OF THE DRAWINGS

FIGURE 1 is a bottom plan view of a sanitary napkin according to the present invention;
FIGURE 2 is a lateral cross-sectional view of the sanitary napkin shown in FIGURE 1 taken along line 2-2 of FIGURE 1;
FIGURE 3 is a bottom plan view of a central absorbent element showing a variant of an adhesive pattern of the present invention;
FIGURE 4 is a bottom plan view of a central absorbent element showing another variant of an adhesive pattern of the present invention;
FIGURE 5 is a bottom plan view of a central absorbent element showing another variant of an adhesive pattern of the present invention;
FIGURE 6 is a bottom plan view of a central absorbent element showing another variant of an adhesive pattern of the present invention;
FIGURE 7 is a bottom plan view of a central absorbent element showing another variant of an adhesive pattern of the present invention;
FIGURE 8 is a bottom plan view of a central absorbent element showing another variant of an adhesive pattern of the present invention;
FIGURE 9 is a bottom plan view of a central absorbent element showing another variant of an adhesive pattern of the present invention;
FIGURE 10 is a bottom plan view of another embodiment of a sanitary napkin according to the present invention; and
FIGURE 11 is a lateral cross-sectional view of the sanitary napkin shown in FIGURE 10 taken along line 11-11 of FIGURE 10;

### DETAILED DESCRIPTION

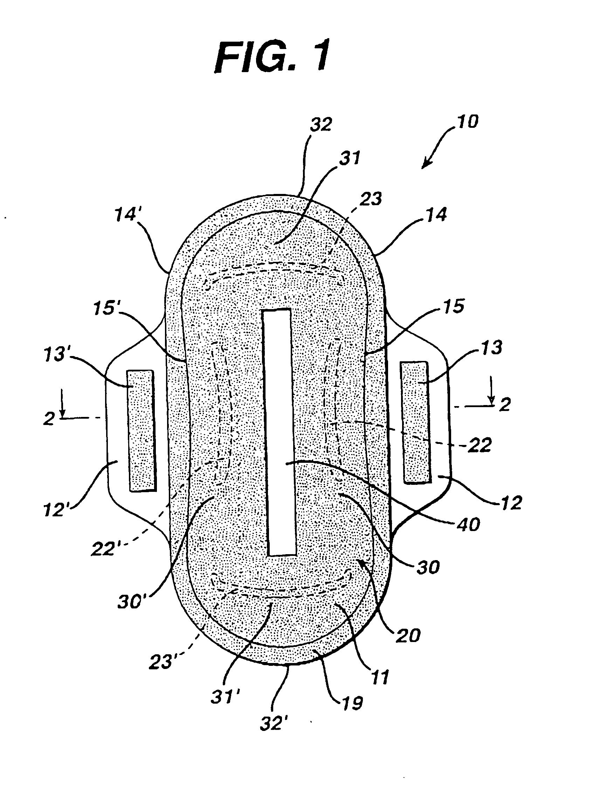

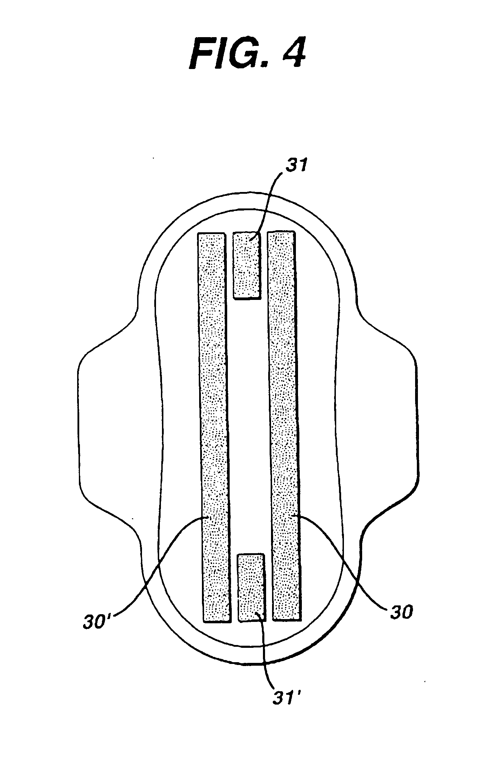

The present invention relates to sanitary absorbent articles such as sanitary napkins, panty liners or incontinence devices and the like. The sanitary absorbent articles have a central absorbent element with opposite longitudinal sides and opposite transverse ends and a pair of side flaps, one flap extending laterally outward from each respective longitudinal side of the central absorbent element. The flaps are formed from a flexible material and are adapted to be folded over a crotch portion of a wearer's undergarment when in use. The absorbent article further includes an adhesive attachment means on a garment faceable surface of the central absorbent element for releasably securing the absorbent article to a wearer's undergarment. The adhesive attachment means comprises a pattern having two longitudinally extending strips and two laterally extending strips. Each longitudinally extending strip is located adjacent to a respective longitudinal side of the central absorbent element and extends substantially from one transverse end region across a central region of the central absorbent element to an opposite transverse end region. Each laterally extending strip is located adjacent to a respective transverse end 32, 32' of the article and extends laterally across at least a portion of the central absorbent element. The two longitudinally extending strips 30, 30' are spaced apart relative to one another. Similarly, the laterally extending strips 31, 31' are spaced apart relative to one another. The longitudinally extending strips may contact the laterally extending strips as shown in FIGURE 1 or may be slightly separated from each other as shown in the embodiments in FIGURES 4-8. In any of these embodiments, it is important that the longitudinally extending strips and the laterally extending strips define a central region intermediate the longitudinally extending strips and the laterally extending strips wherein the central region is non-tacky at room temperature (i.e. about 25° C) and is capable of separating from a wearer's undergarment. In a preferred embodiment the central region intermediate the longitudinally extending strips and the laterally extending strips is substantially free of any adhesive attachment means. Alternatively, the central region may have an adhesive attachment means that is rendered non-tacky at room temperature to permit the center region to separate from a wearer's undergarment when the sanitary absorbent article is in use. In accordance with the preferred embodiment, the adhesive attachment means is inward from both the longitudinal edges and the transverse ends and has a pattern that resembles a "picture frame" wherein the "frame" comprises the adhesive pattern which surrounds a central "picture" region that is capable of separating from a wearer's undergarment when the article is in use.

In a specific example of implementation as illustrated in FIGURE 1, the sanitary absorbent article is a sanitary napkin. As used herein, the term "sanitary napkin" refers to an article which is worn by females in an undergarment adjacent to the pudendal region and which is intended to absorb and contain the various exudates which are discharged from the body (e.g., blood menses, and urine) and which is intended to be discarded after a single use (i.e., it is not intended to be laundered or otherwise restored or reused). The sanitary napkin 10 has an imaginary longitudinal centerline that conceptually divides the sanitary napkin into two substantially symmetrically opposite halves. As used herein, the terminology "longitudinal centerline" refers to an imaginary line, axis or direction of the sanitary napkin 10, which line, axis or direction is typically centered between the longitudinal side edges of the napkin and is generally aligned with the vertical plane which bisects a standing wearer into left and right body halves. The sanitary napkin 10 also has a lateral centerline. The terminology "lateral centerline" refers to an imaginary line, axis or direction generally orthogonal to the longitudinal direction, within the plane of the sanitary napkin 10, and is generally sideways aligned relative to the wearer.

As shown in FIGURE 1, the sanitary napkin 10 comprises a main body represented by central absorbent element 11 and two flaps 12 and 12', one flap extending laterally outward from each respective opposite longitudinal side edge of the central absorbent element 11 in a central portion thereof. Flaps 12, 12' are associated with central absorbent element 11 along respective lines of juncture 15, 15'. The line of juncture may be along the longitudinal edge of the central absorbent element or may be at a location that is juxtaposed to the longitudinal edge and may be in the form of any various curved or straight lines. Each flap has a proximal edge that is coincident with and defined by the line of juncture 15. Each flap extends laterally outward from the line of juncture and terminates as a distal edge. In the embodiment illustrated in FIGURE 1, line of juncture 15 is concave relative to the distal edge, thus the line of juncture 15 curves away from the distal edge. The flaps 12, 12' are located substantially in a central region of each longitudinal edge and are adapted to be folded over a crotch portion of a wearer's undergarment in use. The shape of the flaps 12, 12', as well as the overall shape of the sanitary napkin 10 is not, per se, critical to the invention, provided of course that the sanitary napkin is capable of being comfortably worn in a wearer's undergarment and that the flaps 12, 12' are capable of being readily folded over the crotch portion of the undergarment. Suitable shapes are well known in the art and can be selected by those skilled in the art without undue experimentation. In the embodiment illustrated in FIGURE 1, the flaps are generally trapezoidal in shape and are substantially symmetrically disposed along the longitudinal axis of the sanitary napkin.

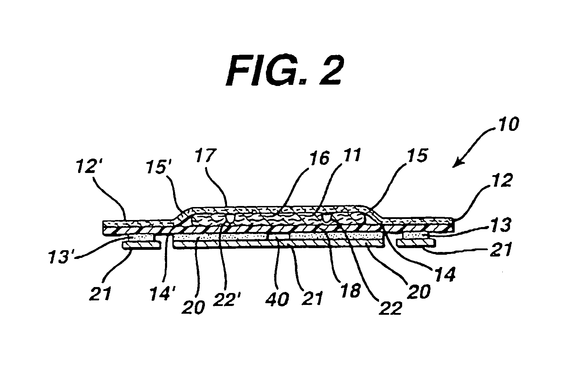

Referring to FIGURE 2, there is shown a cross-sectional view of sanitary napkin 10 taken along line 2-2 of FIGURE 1, which illustrates a preferred embodiment of sanitary napkin 10. As shown in FIGURE 2, central absorbent element 11 comprises topsheet 17, backsheet 18, and absorbent core 16 intermediate topsheet 17 and backsheet 18. Flaps 12, 12' extend laterally outward from central absorbent element 11 and both comprise continuous extensions of topsheet 17 and backsheet 18. Thus, topsheet 17 forms one surface of flap 12 while backsheet 18 forms the other surface, topsheet 17 also forms one surface of central absorbent element 11 while backsheet 18 forms the other surface. In an alternative embodiment, (not shown), the flaps may be formed from materials that are separate and distinct from topsheet 17 and/or backsheet 18 and which are affixed to the central absorbent element.

Topsheet 17 and backsheet 18 are joined at seam 19 (also commonly referred to as a flange seal) around the entire periphery of sanitary napkin 10. The purpose of this seam is to unite the various elements of the sanitary napkin into a unitary structure. Seam 19 may also join topsheet 17 to backsheet 18 immediately adjacent to absorbent core 16 along the proximal edge of the flaps along lines of juncture 15 and 15' (not shown) in FIGURE 2.

As further illustrated in FIGURES 1 and 2, at least a portion of the outer, garment faceable surface of backsheet 18, preferably in a region that is generally in vertical registration with absorbent core 16, is coated with central element adhesive 20 in a pattern substantially as shown. The central element adhesive 20 is adapted to releasably secure the central absorbent element 11 of sanitary napkin 10 within the crotch region of an undergarment. It is preferred that at least a portion of the garment faceable surface of flaps 12, 12', in a region intermediate the proximal edge and the distal edge, also be coated with flap adhesive 13, 13', respectively. Any adhesive used for central element adhesive 20 can also be used as flap adhesive 13, 13'. The flap adhesive 13, 13' is adapted to releasably secure the flaps to an underside of the crotch region of an undergarment. Any of the conventional adhesives or glues that are used in the art for releasably securing sanitary napkins to undergarments is suitable for use herein, with pressure-sensitive adhesives being preferred. Suitable adhesives include, but are not limited to Century A-305IV manufactured by the Century Adhesives Corporation, Instant LOK 34-2823 manufactured by National Starch Company, and the like. The central element adhesive 20 and flap adhesive 13 are typically covered by respective release liners 21. The release liners keep the adhesive from drying out and keep it from sticking to extraneous surfaces prior to use. Any commercially available release liner material commonly used for such purposes can be used herein. Non-limiting examples of suitable release liners are BL30MG-A SILOXE1/0 and BL 30 MG-A SILOX 4/P/O, both of which are manufactured by the Akrosil Corporation.

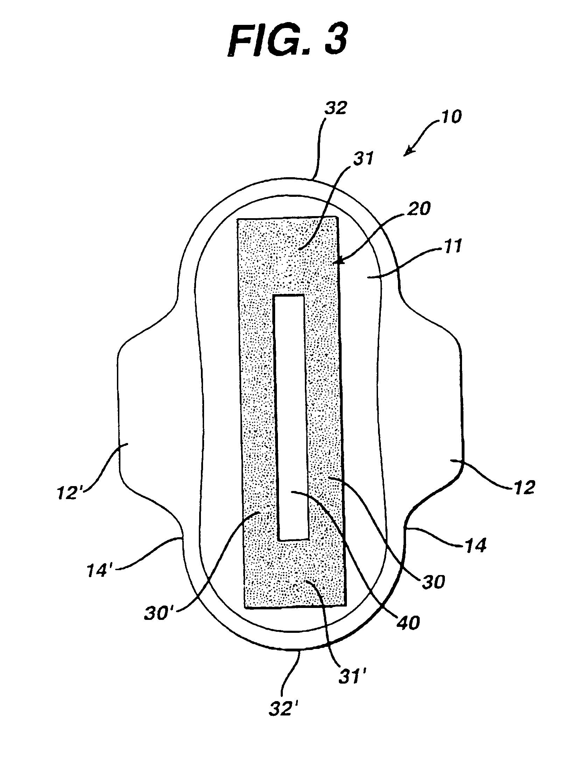

The central element adhesive 20 has a pattern that includes two longitudinally extending strips 30, 30' and two laterally extending strips 31, 31'. Each longitudinally extending strip is located adjacent to a respective longitudinal side 14, 14' of the central absorbent element 20 and extends generally parallel to the longitudinal centerline from one transverse end region across a central region of the central absorbent element 20 to an opposite transverse end region. In a preferred embodiment as shown in FIGURE 3, each longitudinally extending strip is located inward from each respective longitudinal side 14, 14'. Each laterally extending strip 31, 31' is located adjacent to a respective transverse end region of the napkin and extends across at least a portion of the width of the central absorbent element in a direction generally parallel to the lateral centerline of the napkin. In a preferred embodiment as shown in FIGURE 3, each laterally extending strip is located inward from each respective transverse end. The two longitudinally extending strips 30, 30' are spaced apart relative to one another. Similarly, the laterally extending strips 31, 31' are spaced apart relative to one another. As shown in Figure 1, the two longitudinally extending strips 30, 30' contact the two laterally extending strips 31, 31' to form a complete rectangular adhesive pattern which fully encloses a central region 40. Thus, together the longitudinally extending strips 30, 30' and the laterally extending strips 31, 31'define central region 40 which is intermediate the longitudinally extending strips 30, 30' and the laterally extending strips 31, 31'. The central region 40 is non-tacky at room temperature and thus does not include an adhesive attachment means to releasably secure that region of the central absorbent element 11 to a wearer's undergarment. It is an important feature of the present invention that the central region 40 be non-tacky at room temperature and is thus capable of separating from a wearer's undergarment when the sanitary napkin 10 is in use. The central region 40 permits the center portion of the sanitary napkin to deform upward when subjected to laterally compressive forces and enables the napkin to better conform to the wearer's body. Thus, the central element adhesive 20 has a pattern that generally resembles a "picture frame" wherein the longitudinally extending strips 30, 30' and the laterally extending strips 31, 31' define the frame which surrounds a central region 40 (picture) that is capable of separating from a wearer's undergarment when the article is in use.

In a preferred embodiment of the invention, the central region 40 has a length of from about 10 cm to about 12 cm, more preferably from about 10.5 cm to 11.5 cm and most preferably about 11 cm. The central region 40 generally has a width of from about 1 cm to about 5 cm, preferably about 1.5 cm to about 4 cm and most preferably from about 2 cm to about 3 cm. The total length of the central element adhesive 20 can vary widely, depending of course on the length of the central absorbent element 11 and is generally from about 18 cm to 20 cm for a sanitary napkin having a length of about 22 cm. For sanitary napkins having longer lengths, such as sanitary napkins designed for overnight use, it is preferred that the lengths of the lateral strips 31, 31' are extended to cover the additional length of material in the transverse end region of the sanitary napkin rather than extend the longitudinal strips 30, 30'. Accordingly, the length of the center region 40 is preferably maintained within the above-described ranges. The expedient of extending the length of the lateral strips 31, 31' while maintaining a central region length of between about 10 cm to about 12 cm has been found to provide enhanced conformance to the wearer's body and better stability and resistance to deformation in the transverse end regions of the napkin and thereby prevent bunching or roping of the napkin.

The adhesive on the longitudinally extending strips 30, 30', as well as the laterally extending strips 31, 31' may be continuous throughout their respective lengths or widths or may be intermittent, provided of course that there is sufficient adhesive present in these regions to maintain a substantial portion of the longitudinal side edges and transverse end regions of the central absorbent element in adhesive contact with the wearer's undergarment. The spacing between the intermittently applied adhesive should not be so great so as to permit the sanitary napkin to separate from the undergarment in use and create a folding axis within the sanitary napkin. The adhesive may be applied directly onto the backsheet 18 or may be applied indirectly. A convenient way to indirectly apply the adhesive to the backsheet is to hot melt the adhesive onto a release liner and then bring the adhesive coated release liner into contact with the backsheet 18 of the sanitary napkin 10 whereby the adhesive preferentially transfers to the surface of the backsheet.

Referring again to Figures 1 and 2, the sanitary napkin 10 includes a topsheet 17 that is in close proximity to the skin of the user when sanitary napkin 10 is in use. Topsheet 17 is liquid permeable and is preferably compliant, soft feeling, and non-irritating to the user's skin. It can be made from any of the materials conventional for this type of use. Non-limiting examples of suitable materials that can be used as topsheet 17 are woven and nonwoven polyester, polypropylene, nylon, and/or rayon fabrics and apertured thermo-plastic films. Apertured formed films are preferred for topsheet 17 because they are pervious to liquids and yet non-absorbent. Thus, the surface of the formed film that is in contact with the body remains dry and is more comfortable to the wearer. In the embodiment illustrated in FIGS 1 and 2, topsheet 17 serves as a topsheet for flaps 12 and 12'.

The sanitary napkin 10 also includes a backsheet 18. Backsheet 18 is impervious to liquids and thus prevents menstrual fluid from soiling the clothing of the user. Any conventional materials used in the art for such purpose are suitable for use herein. Suitable materials include embossed or non-embossed polymeric films such as polyethylene films, polypropylene films and the like. In the embodiment illustrated in FIGURES 1 and 2, backsheet 18 serves as a backsheet for flaps 12 and 12'.

Absorbent core 16 provides the means for absorbing menstrual fluid. Absorbent core 16 is generally compressible, comfortable and non-irritating to the user's skin. It can comprise any material used in the art for such purpose. Examples include comminuted wood pulp that is generally referred to as air-felt, creped cellulose wadding, absorbent foams, absorbent sponges, absorbent hydrogel materials, polymeric fibers, or any equivalent material or combinations of materials. Absorbent core 16 will generally have an absorbent capacity sufficient to absorb the anticipated total amount of menstrual fluid.

Absorbent core 16 is preferably narrow, i.e. less than about 75 mm and is preferably thin, i.e. less than about 5 mm in caliper, preferably less than 4 mm and most preferably less than 3 mm in caliper. It has been found that a sanitary napkin having a narrow, thin absorbent core is extremely comfortable to the user. It should be noted that a narrow, thin absorbent core 16 is most effective because the overall configuration and use of sanitary napkin 10 results in a central portion of the central absorbent element 11 that is capable of easily separating from a wearer's undergarment to better maintain a close proximity to the body while the transverse end regions are secured to the wearer's undergarment to better resist bunching, folding or twisting of the central absorbent element 11. Such proximity of central absorbent element 11 places it precisely where it should be: very near the body at the vaginal opening. Absorbent core 16 can then absorb the vast majority of the menstrual fluid (menses) before it has an opportunity to flow along the central absorbent element 11. In a preferred embodiment, the absorbent core 16 has a central region with a caliper that exceeds the caliper of the peripheral edge regions. These shaped absorbent cores are commonly referred to as a "raised center" core. The thicker caliper center region is adapted to be located adjacent to the wearer's vaginal orifice to better maintain close proximity thereto. The thinner caliper edge margins are capable of easily deforming when subjected to any laterally compressive forces that may be imparted by the wearer's thighs. The combination of the present adhesive pattern on a raised center absorbent core enhances the ability of the napkin to maintain body contact without any discomfort to the wearer. In accordance with this embodiment, the caliper of the center region of the absorbent core is from about 3 cm to about 8 cm and the caliper of the surrounding peripheral edge margins is from about 2 cm to about 5 cm, with the proviso that the caliper of the center region exceeds the caliper of the peripheral edge margins by at least 1 cm, preferably by at least 2 cm.

The central absorbent element 11 may be provided with one or more longitudinally extending hinges 22, 22', preferably vertically aligned with the longitudinally extending strips 30, 30', to enable the central absorbent element to preferentially bend in a pre-determined region. The longitudinally extending hinges may be in the form of compressed, embossed channels that are slightly inward from the longitudinal side edges 15, 15' of the central absorbent element. The compressed, embossed channels are formed by highly densifying a portion of the absorbent core 16, optionally with the topsheet and/or backsheet to form a defined valley within the absorbent core. The hinges 22, 22' may extend along the entire length of the central absorbent element or preferably are located solely in a center region of the central absorbent element, one hinge being located adjacent to each respective longitudinal side edge 15, 15'. In a most preferred embodiment, the hinges 22, 22'comprise embossed channels that are arcuate, having a concave inward orientation with respect to the longitudinal side edges 15, 15' to create the impression of an hour-glass shape.

Alternatively or in addition to the above longitudinally extending hinges, the central absorbent element 11 may be provided with one or more laterally extending hinges 23, 23', preferably vertically aligned with the laterally extending strips 31, 31' in the transverse end regions of the sanitary napkin. The laterally extending hinges 23, 23' permit the sanitary napkin to preferentially laterally fold to allow the end regions of the napkin to conform to the wearer's body. The laterally extending hinges are preferably in the form of compressed, embossed channels that are slightly inward from the longitudinal side edges 15, 15' and transverse ends of the central absorbent element. The laterally extending embossed channels provide lateral stiffness to the transverse end regions that enables these regions to resist deformation due to laterally compressive forces.

As shown in FIGURES 1 and 2, topsheet 17 is secured to backsheet along seam 19, also called flange seal. Seam 19 can be formed by any means commonly used in the art for this purpose such as by gluing, crimping, or heat-sealing. Seam 19 is illustrated extending completely around the periphery of sanitary napkin 20; this is a suitable embodiment for ease of construction. (Other means of uniting the various elements can be used.)

The sanitary napkin shown in FIGURES 1 and 2 has a curvilinear line of juncture 15. As shown absorbent core 16 is generally hourglass shaped so as to conform generally to the wearer's thighs. Thus, the line of juncture 15 between the flap 12 and the absorbent core 16 is curvilinear. It should be noted that lines of juncture 15 and 15' are the lines along which flaps 12 and 12' are associated with the absorbent core (represented by central absorbent element 11); as such they represent lines of demarcation between the absorbent core and the flaps.

The sanitary napkin of the present invention, such as the one illustrated in FIGURES 1 and 2, is utilized by removing the release liners 21 and thereafter placing the sanitary napkin in a panty such that the center region of central absorbent element 11 is placed in crotch portion of the panty with one end of central absorbent element 11 extending towards the front section of the panty and the other end towards the back section and with the backsheet 18 in contact with the inner surface of center crotch portion of the panty. Central element adhesive 20 maintains central absorbent element 11 in position. The distal portions of flaps 12 and 12' are folded around, respectively, the opposite side edges of the crotch portion of the panty. Flap adhesive 13 and 13' releasably secure flaps 12 and 12' in such position, thus, flaps 12 and 12' are each folded over themselves with a portion of the panty, including the side edges interposed therebetween.

Referring now to FIGURE 3, there is shown another embodiment of the sanitary napkin 10 of the present invention. Flaps 12, 12' extend laterally outward from a central region of central absorbent element 11. Central adhesive element 20 has longitudinally extending strips 30, 30' and laterally extending strips 31, 31'. The longitudinally extending strips 30, 30' and laterally extending strips 31, 31' are spaced inward from respective longitudinal sides 14, 14' and transverse ends 32, 32'. The longitudinally extending strips 30, 30' and laterally extending strips 31, 31' contact one another to define a fully enclosed central region 40.

FIGURE 4 shows another embodiment of the invention wherein the longitudinally extending strips 30, 30' and the laterally extending strips 31, 31' are separated one from the other and together define a central region 40. The longitudinally extending strips 30, 30' are longitudinally co-terminous with the laterally extending strips 31, 31', and the laterally extending strips 31, 31' are substantially intermediate the longitudinally extending strips 30, 30'.

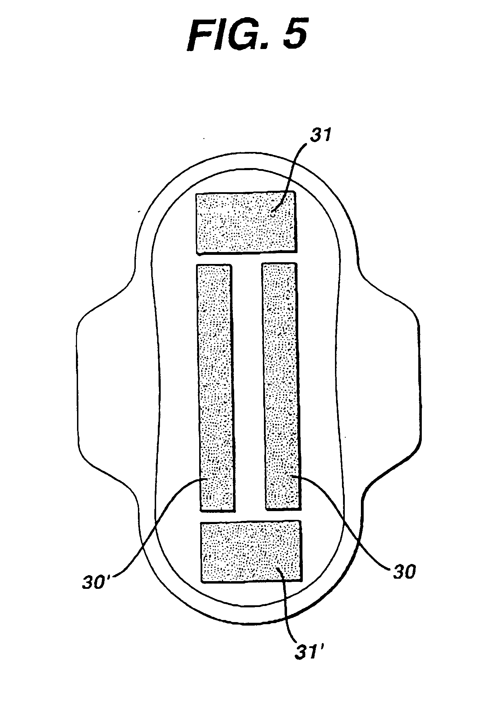

FIGURE 5 shows another embodiment of the present invention wherein the longitudinally extending strips 30, 30' and the laterally extending strips 31, 31' are separated one from the other and together define a central region 40. In accordance with this embodiment, the longitudinally extending strips 30, 30' terminate inward from the longitudinally extending strips 30, 30' and are thus intermediate the laterally extending strips 31, 31'.

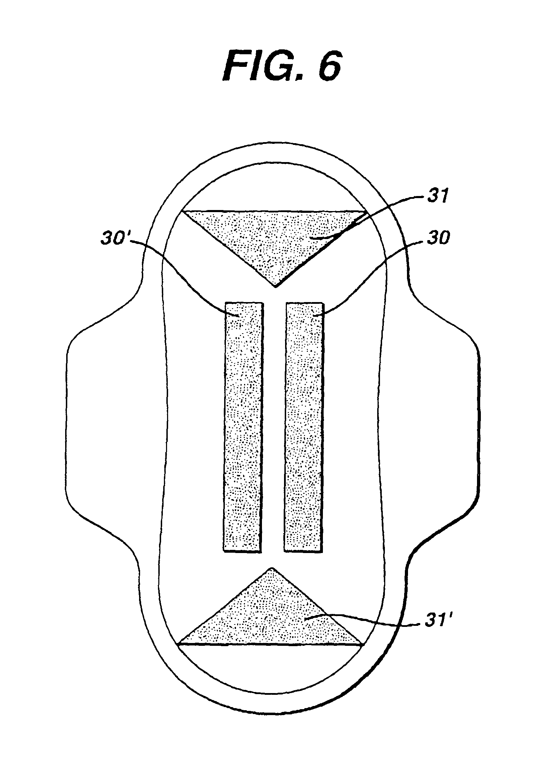

FIGURE 6 shows another embodiment of the present invention wherein the longitudinally extending strips 30, 30' and the laterally extending strips 31, 31' are separated one from the other and together define a central region 40. In accordance with this embodiment, the laterally extending strips 31, 31' are in the general shape of a triangle.

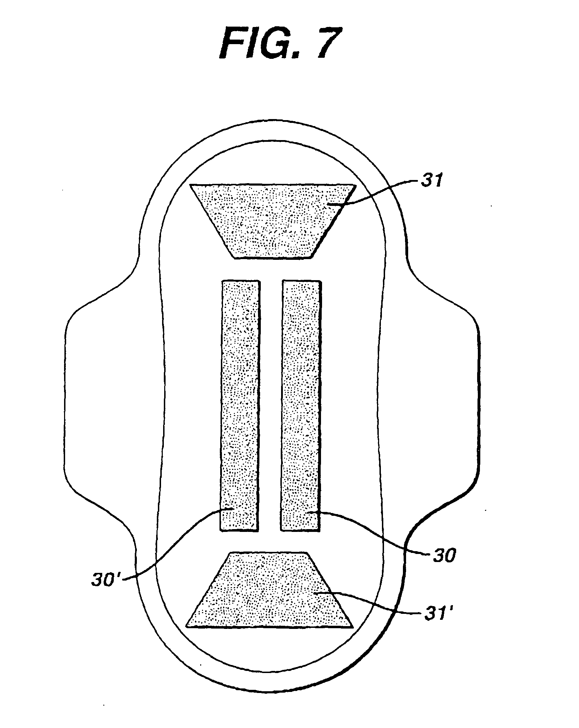

FIGURE 7 shows another embodiment of the present invention wherein the longitudinally extending strips 30, 30' and the laterally extending strips 31, 31' are separated one from the other and together define a central region 40. In accordance with this embodiment, the laterally extending strips 31, 31' are in the general shape of a trapezoid.

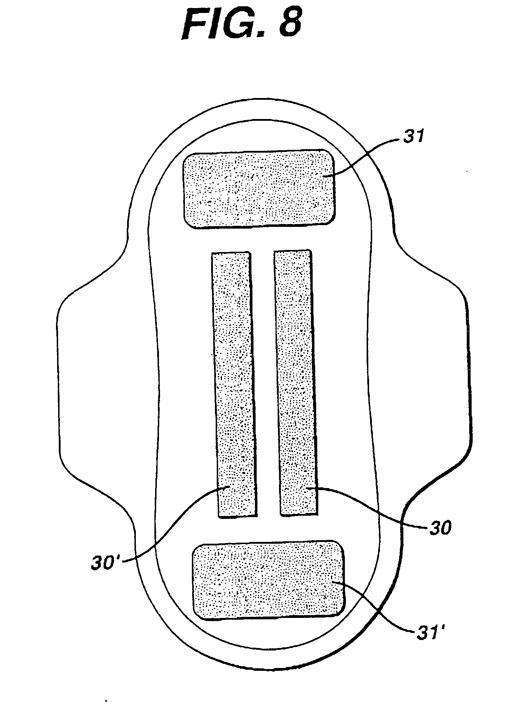

FIGURE 8 shows another embodiment of the present invention wherein the longitudinally extending strips 30, 30' and the laterally extending strips 31, 31' are separated one from the other and together define a central region 40. In accordance with this embodiment, the longitudinally extending strips 30, 30' terminate inward from the longitudinally extending strips 30, 30' and the laterally extending strips 31, 31' have a width that extends laterally beyond the longitudinal sides of the longitudinally extending strips 30, 30'.

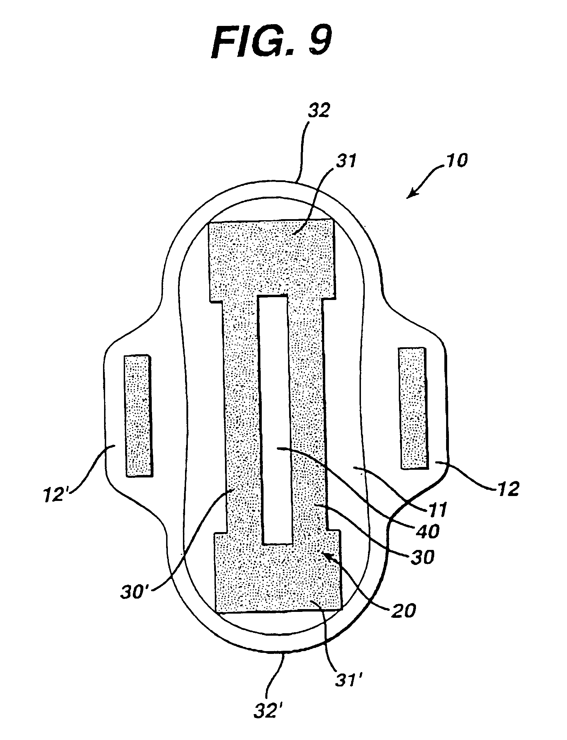

Referring now to FIGURE 9, there is shown another embodiment of the sanitary napkin 10 of the present invention. Flaps 12, 12' extend laterally outward from a central region of central absorbent element 11. Central adhesive element 20 has longitudinally extending strips 30, 30' and laterally extending strips 31, 31'. Both the longitudinally extending strips 30, 30' and laterally extending strips 31, 31' are spaced inward from respective longitudinal sides 14, 14' and transverse ends 32, 32'. The longitudinally extending strips 30, 30' and laterally extending strips 31, 31' contact one another to define a fully enclosed central region 40. In accordance with this embodiment, the laterally extending strips 31, 31' have a width that extends laterally beyond the longitudinal sides of the longitudinally extending strips 30, 30'. The wider laterally extending strips 31, 31' have been found to provide additional stability to the end regions of the sanitary napkin and thus prevent or substantially inhibit bunching or roping of the sanitary napkin.

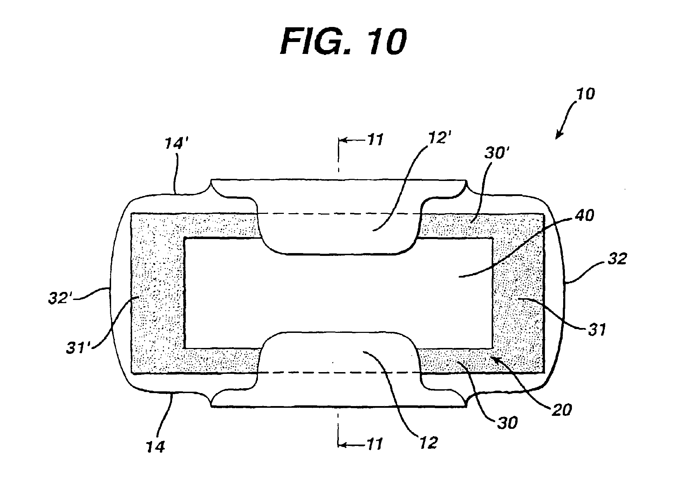

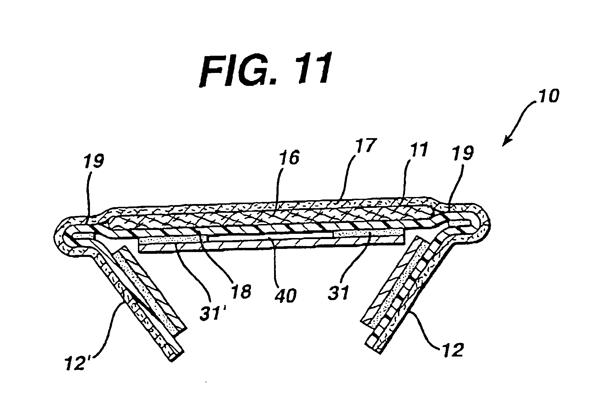

Referring now to FIGURES 10 and 11, there is shown another embodiment of the sanitary napkin 10 of the present invention. In accordance with this embodiment, flaps 12, 12' are affixed to a garment faceable surface of the sanitary napkin 10 in a central region of central absorbent element 11. Central adhesive element 20 has longitudinally extending strips 30, 30' and laterally extending strips 31, 31'. The longitudinally extending strips 30, 30' and laterally extending strips 31, 31' are spaced inward from respective longitudinal sides 14, 14' and transverse ends 32, 32'. The longitudinally extending strips 30, 30' and laterally extending strips 31, 31' contact one another to define a fully enclosed central region 40.

Referring to FIGURE 11, there is shown a cross-sectional view of sanitary napkin 10 taken along line 11-11 of FIGURE 10, which illustrates a preferred embodiment of sanitary napkin 10. As shown in FIGURE 11, central absorbent element 11 comprises topsheet 17, backsheet 18, and absorbent core 16 intermediate topsheet 17 and backsheet 18. Flaps 12, 12' extend laterally inward from a respective opposite longitudinal side 14, 14' of central absorbent element 11 and both comprise continuous extensions of topsheet 17 and backsheet 18 that have been affixed to the backsheet 18, inward from the longitudinal sides 14, 14', respectively. Thus, topsheet 17 forms one surface of flap 12 while backsheet 18 forms the other surface, topsheet 17 also forms one surface of central absorbent element 11 while backsheet 18 forms the other surface. In an alternative embodiment, (not shown), the flaps may be formed from materials that are separate and distinct from topsheet 17 and/or backsheet 18 and which are affixed to the central absorbent element. Topsheet 17 and backsheet 18 are joined at seam 19 (also commonly referred to as a flange seal) around the entire periphery of sanitary napkin 10. The purpose of this seam is to unite the various elements of the sanitary napkin into a unitary structure.

## Claims

1. A sanitary absorbent article (10) comprising a central absorbent element (11) with opposite longitudinal sides (14, 14') and opposite transverse ends (32, 32'), the absorbent article including a pair of flexible side flaps (12, 12'), one flap (12, 12') extending laterally outward from each respective longitudinal side (14, 14') of the absorbent element (10), the flaps (12, 12') being adapted to be folded over a crotch portion of a wearer's undergarment when in use, the absorbent article (10) further including a adhesive attachment means on a garment faceable surface for securing the article to the undergarment **characterized in that**
the adhesive attachment means have a pattern including two longitudinally extending strips (30, 30'), one strip (30, 30') being located adjacent to each respective longitudinal side (14, 14') of the article and two laterally extending strips (31, 31'), one strip (31, 31') being located adjacent to each respective transverse end (32, 32') of the article said longitudinal extending strips (30, 30') and said laterally extending strips (31, 31') substantially surrounding a center region (40) that is non-tacky at room temperature and is incapable of adhesively securing that portion of the garment faceable surface of the article to the wearer's undergarment in use, wherein said center region (40) has a length of from 10 cm to 12cm.

2. A sanitary absorbent article as defined in claim 1, the two longitudinally extending strips (30, 30') contact the two laterally extending strips (31, 31') to fully enclose the center region (40).

3. A sanitary absorbent article as defined in claim 1, wherein said sanitary absorbent article is a sanitary napkin.

4. A sanitary absorbent article as defined in claim 1, wherein the two longitudinally extending strips (30, 30') are separated from and do not contact the two laterally extending strips (31, 31').

5. A sanitary absorbent article as defined in claim 4, wherein the two longitudinally extending strips (30, 30') are intermediate the two laterally extending strips (31, 31').

6. A sanitary absorbent article as defined in claim 5, wherein the two laterally extending strips (31, 31') are intermediate the two longitudinally extending strips (30, 30').

7. A sanitary absorbent article as defined in claim 1, wherein each longitudinally extending strips (30, 30') is inward from the each respective longitudinal side (14, 14').

## Patentansprüche

1. Absorbierender Hygieneartikel (10), umfassend ein zentrales absorbierendes Element (11) mit einander gegenüberliegenden longitudinalen Seiten (14, 14') und einander gegenüberliegenden transversalen Enden (32, 32'), wobei der absorbierende Artikel ein Paar flexibler Seitenflügel (12, 12') einschließt, und ein Flügel (12, 12') sich seitlich nach außen von jeder entsprechenden longitudinalen Seite (14, 14') des absorbierenden Elements (10) aus erstreckt, wobei Flügel (12, 12') dafür geeignet sind, beim Gebrauch über einen Genitalbereich der Unterbekleidung der Trägerin gefaltet zu werden, und wobei der absorbierende Artikel (10) weiterhin ein Klebebefestigungsmittel auf einer der Kleidung zugewandten Oberfläche für die Befestigung des Artikels an der Unterbekleidung umfasst, **dadurch gekennzeichnet, dass**
das Klebebefestigungsmittel ein Muster aufweist, umfassend zwei sich longitudinal erstreckende Streifen (30, 30'), wobei ein Streifen (30, 30') benachbart zu jeder entsprechenden longitudinalen Seite (14, 14') des Artikels positioniert ist, und zwei sich seitlich erstreckende Streifen (31, 31'), wobei ein Streifen (31, 31') benachbart zu jedem jeweiligen transversalen Ende (32, 32') des Artikels positioniert ist, wobei die sich longitudinal erstreckenden Streifen (30, 30') und die sich seitlich erstreckenden Streifen (31, 31') im Wesentlichen einen zentralen Bereich (40) umgeben, der bei Raumtemperatur nicht klebend ist und nicht in der Lage ist, jenen Bereich der der Kleidung zugewandten Oberfläche des Artikels beim Gebrauch klebend an der Unterbekleidung der Trägerin zu befestigen, wobei der zentrale Bereich (40) eine Länge im Bereich von 10 cm bis 12 cm aufweist.

2. Absorbierender Hygieneartikel, wie in Anspruch 1 definiert, wobei die zwei sich longitudinal erstreckenden Streifen (30, 30') die zwei sich seitlich erstreckenden Streifen (31, 31') kontaktieren, um den zentralen Bereich (40) vollständig zu umgeben.

3. Absorbierender Hygieneartikel, wie in Anspruch 1 definiert, **dadurch gekennzeichnet, dass** der absorbierende Hygieneartikel eine Damenbinde ist.

4. Absorbierender Hygieneartikel, wie in Anspruch 1 definiert, **dadurch gekennzeichnet, dass** die zwei sich longitudinal erstreckenden Streifen (30, 30') von den zwei sich seitlich erstreckenden Streifen (31, 31') getrennt sind und diese nicht kontaktieren.

5. Absorbierender Hygieneartikel, wie in Anspruch 4 definiert, **dadurch gekennzeichnet, dass** die zwei sich longitudinal erstreckenden Streifen (30, 30') sich zwischen den zwei sich seitlich erstreckenden Streifen (31, 31') befinden.

6. Absorbierender Hygieneartikel, wie in Anspruch 5 definiert, **dadurch gekennzeichnet, dass** die zwei sich seitlich erstreckenden Streifen (31, 31') sich zwischen den zwei sich longitudinal erstreckenden Streifen (30, 30') befinden.

7. Absorbierender Hygieneartikel, wie in Anspruch 1 definiert, **dadurch gekennzeichnet, dass** jeder der sich longitudinal erstreckenden Streifen (30, 30') sich einwärts von jeder der jeweiligen longitudinalen Seite (14, 14') aus befindet.

## Revendications

1. Article hygiénique absorbant (10) comportant un élément absorbant central (11) ayant des côtés longitudinaux opposés (14,14') et des extrémités transversales opposées (32, 32'), l'article absorbant comprenant une paire de volets latéraux souples (12, 12'), un volet (12, 12') s'étendant latéralement vers l'extérieur à partir de chaque côté longitudinal respectif (14, 14') de l'élément absorbant (10), les volets (12, 12') étant adaptés pour être repliés sur une partie de crochets d'un sous-vêtement d'utilisateur en utilisation, l'article absorbant (10) comprenant en outre des moyens de fixation adhésifs sur une surface pouvant être mise en vis-à-vis d'un vêtement pour fixer l'article sur le sous-vêtement,
**caractérisé en ce que**
les moyens de fixation adhésifs ont un motif comprenant deux bandes s'étendant longitudinalement (30, 30'), une première bande (30, 30') étant positionnée adjacente à chaque côté longitudinal respectif (14, 14') de l'article, et deux bandes s'étendant latéralement (31, 31'), une bande (31, 31') étant située adjacente à chaque extrémité transversale respective (32, 32') de l'article, lesdites bandes s'étendant longitudinalement (30, 30') et lesdites bandes s'étendant latéralement (31, 31') entourant sensiblement une zone centrale (40) qui est non collante à température ambiante, et qui est incapable de fixer de manière adhésive cette partie de la surface pouvant être mise en vis-à-vis d'un vêtement de l'article sur le sous-vêtement du porteur en utilisation, dans lequel ladite zone centrale (40) a une longueur de 10 cm à 12 cm.

2. Article hygiénique absorbant selon la revendication 1, dans lequel les deux bandes s'étendant longitudinalement (30, 30') viennent en contact avec les deux bandes s'étendant latéralement (31, 31') pour entourer complètement la zone centrale (40).

3. Article hygiénique absorbant selon la revendication 1, dans lequel ledit article hygiénique absorbant est une serviette hygiénique.

4. Article hygiénique absorbant selon la revendication 1, dans lequel les deux bandes s'étendant longitudinalement (30, 30') sont séparées des deux bandes s'étendant latéralement (31, 31'), et ne viennent pas en contact avec celles-ci.

5. Article hygiénique absorbant selon la revendication 4, dans lequel les deux bandes s'étendant longitudinalement (30, 30') se trouvent entre les deux bandes s'étendant latéralement (31, 31').

6. Article hygiénique absorbant selon la revendication 5, dans lequel les deux bandes s'étendant latéralement (31, 31') se trouvent entre les deux bandes s'étendant longitudinalement (30, 30').

7. Article hygiénique absorbant selon la revendication 1, dans lequel chaque bande s'étendant longitudinalement (30, 30') est vers l'intérieur par rapport à chaque côté longitudinal respectif (14, 14').
